# FASCICULE DE BREVET EUROPEEN

(11) **EP 2 413 879 B1**
(45) Date de publication et mention de la délivrance du brevet: **16.08.2017**
(21) Numéro de dépôt: 09784369.2
(22) Date de dépôt: 31.03.2009
(51) Int. Cl.: A61K 8/19, A61K 8/41, A61K 8/73, A61Q 5/12

(54) **COMPOSITION CAPILLAIRE DE TRAITEMENT DES CHEVEUX**
KAPILLARZUSAMMENSETZUNG ZUR HAARBEHANDLUNG
CAPILLARY COMPOSITION FOR TREATING HAIR

(43) Date de publication de la demande: 08.02.2012
(73) Titulaire: L'Oréal, 75008 Paris (FR)
(72) Inventeur: KHENNICHE, Samira, F-92110 Clichy (FR); ROLLAT-CORVOL, Isabelle, F-75017 Paris (FR); BOUREL, Sophie, F-92150 Suresnes (FR)
(74) Mandataire: Nony
(86) Numéro de dépôt international: PCT/FR2009/050550
(87) Numéro de publication internationale: WO 2010/112682

(56) Documents cités:
- DE-A1-102005 060 435
- US-A- 4 874 554
- US-A1- 2002 012 697
- US-A1- 2006 234 886
- US-A1- 2007 140 985
- DATABASE GNPD [en ligne] MINTEL 01 Mai 2006 'Messy Mud' Database accession no. 530333
- DATABASE GNPD [en ligne] MINTEL 01 Décembre 2008 'Rough Up Modelling Clay' Database accession no. 1023973

## Description

La présente invention est relative au traitement des cheveux humains.

Les cheveux peuvent être altérés par des traitements chimiques tels que la coloration, la permanente, ou par des sollicitations mécaniques telles que le démêlage, le brushing. Les propriétés mécaniques, morphologiques et physicochimiques de la surface des cheveux, et notamment de la cuticule, couche externe du cheveu avec une structure en écailles, s'en trouvent modifiées. En particulier, au cours de ces traitements ou sollicitations, les écailles de la cuticule se soulèvent et voient leurs bordures, normalement régulières, devenir dentelées. Ces détériorations peuvent avoir plusieurs conséquences. D'une part, les cheveux sont moins lisses et moins facilement démêlables. D'autre part, les actifs d'un produit de traitement des cheveux, par exemple d'un conditionneur, risquent d'être déposés de façon hétérogène sur le cheveu. Or, il peut s'avérer souhaitable de déposer les actifs de soin de manière homogène sur tout le cheveu.

Il est connu de soigner des cheveux abîmés en appliquant sur ceux-ci un produit de soin comprenant par exemple des polymères spécifiques tels que des silicones ou des polymères à charges cationiques. Cependant, l'amélioration des cheveux ainsi traités n'est que provisoire car une fois débarrassés du produit de soin, par exemple après un ou plusieurs lavages, les cheveux retrouvent leur état d'origine.

Il existe un besoin de compositions, de procédés et de kits permettant de traiter durablement les cheveux, en particulier ceux abîmés en surface.

L'invention vise entre autres à répondre à ce besoin et elle y parvient grâce à un procédé de traitement des cheveux mettant en oeuvre une composition capillaire comprenant au moins des particules de pierre ponce, un ou plusieurs tensioactifs cationiques comportant au moins une fonction ester et un ou plusieurs polymères non siliconés.

Un premier objet de l'invention est donc une composition capillaire comprenant au moins des particules de pierre ponce, un ou plusieurs tensioactifs cationiques comportant au moins une fonction ester et un ou plusieurs polymères non siliconés, choisis parmi les polymères épaississants à motifs sucres. Un second objet de l'invention est un procédé de traitement des cheveux comportant au moins l'étape consistant à mettre en contact les cheveux avec une composition capillaire comme indiquée ci-dessus.

Comme il ressort des exemples ci-après, le traitement des fibres kératiniques, et notamment des cheveux, avec une composition conforme à l'invention permet d'apporter du soin de manière homogène à la fibre kératinique sans l'altérer en uniformisant le dépôt des agents traitants. On obtient ainsi un excellent lissage des fibres kératiniques et un volume de la chevelure maitrisé. Ces effets sont d'autant plus remarquables que les cheveux sont sensibilisés et/ou épais.

Ces effets sont durables et résistent notamment au(x) shampooing(s).

Le procédé peut comporter également l'étape consistant à peigner et/ou rincer les cheveux à l'issue dudit traitement.

Le procédé peut être également avantageusement mis en oeuvre pour lisser les cheveux.

L'invention peut également permettre de préparer les cheveux à un post-traitement capillaire tel que l'application d'un conditionneur, d'une coloration, d'une permanente, d'un produit de défrisage, d'un produit de décoloration ou autre.

Le procédé peut ainsi comporter l'étape consistant à soumettre les cheveux à un post-traitement, après traitement à l'aide de la composition capillaire de l'invention, le post-traitement étant choisi parmi l'application d'un conditionneur, d'une permanente, d'un défrisant, d'un produit de coloration ou de décoloration des cheveux, cette liste n'étant pas limitative.

Le traitement des cheveux qui est opéré en mettant en oeuvre l'invention peut être plus ou moins important, en fonction de l'état initial des cheveux et/ou du résultat recherché. Ce traitement peut notamment avoir pour effet d'inclure une élimination des hétérogénéités figurant en surface des cheveux, notamment *via* une action que l'on peut qualifier d'abrasive et, de ce fait, homogénéiser la surface externe des cheveux.

Ce type d'abrasion peut être relativement doux et/ou de courte durée, de manière à éviter une cassure des cheveux, lors du traitement ou de sollicitations mécaniques ultérieures telles que le coiffage, par exemple.

Grâce à l'invention, les cheveux peuvent être visiblement plus lisses et l'effet du traitement est durable.

Sans être lié par une quelconque théorie on peut penser que grâce à l'invention, les cheveux peuvent être débarrassés de dépôts éventuellement présents à leur surface avant l'abrasion et les bordures des écailles de la cuticule peuvent être rendues plus régulières, ce qui permet une efficacité accrue du ou des tensioactifs cationiques comportant au moins une fonction ester.

De plus, après le traitement, des produits destinés à renforcer certaines propriétés des cheveux ou à modifier leur aspect peuvent pénétrer plus facilement et profondément dans les cheveux ainsi traités.

### Composition capillaire

### Particules de pierre ponce

La pierre ponce (nom INCI : pumice) est d'origine volcanique. Elle se forme à des températures d'environ 500 à 600 °C à partir de la lave projetée en l'air qui se refroidit dans sa chute et dont le dégazage entraîne la formation de bulles dont résultent une densité faible et une porosité importante.

La pierre ponce est formée de fragments de rhyolite, de dacite ou d'andésite. Elle est considérée comme un verre car elle n'a pas de structure cristalline.

Les particules de pierre ponce sont des particules solides abrasives. En particulier, elles peuvent avoir une dureté supérieure ou égale à celle des cheveux, allant de 3 à 10 Mohs, voire supérieure ou égale à 4, par exemple supérieure ou égale à 5, et en particulier allant de 5 à 5,5 sur l'échelle de Mohs.

Les particules de pierre ponce peuvent avoir un diamètre moyen en volume inférieur ou égal à 500 µm, de préférence compris entre 50 et 500 µm, mieux inférieur ou égal à 300 µm, par exemple compris entre 100 et 300 µm.

Suivant la gamme de particules mises en oeuvre, le diamètre moyen en volume peut être déterminé par utilisation de tamis ou par granulométrie laser.

Il peut notamment s'agir d'une poudre de pierre ponce commercialisée sous le nom de PONCE 0 ½ D par la société EYRAUD, de diamètre moyen D [4,3] (diamètre moyen pondéré en volume) d'environ 140 µm mesuré par diffraction laser.

Il peut également s'agir d'une poudre de pierre ponce décontaminée commercialisée sous la référence 0-D PONCE par la Société EYRAUD, de diamètre moyen en volume inférieur à 125 µm, ou encore d'une poudre de pierre ponce commercialisée sous la référence 2B D par la Société EYRAUD, de diamètre moyen en volume allant de 100 à 500 µm.

La composition capillaire conforme à l'invention peut comporter des particules de pierre ponce en une teneur allant de 0,1 % à 35 % en poids, notamment de 5 % à 30 % en poids, en particulier de 10 % à 25 % en poids, par exemple de 15 % à 20 % en poids, mieux de 18 % à 20 % en poids par rapport au poids total de la composition.

### Tensioactifs cationiques comportant au moins une fonction ester

Il s'agit des sels d'ammonium quaternaire contenant au moins une fonction ester de formule (I) suivante : 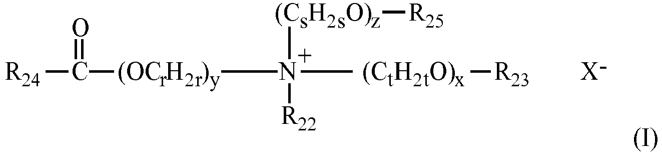 dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 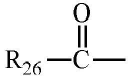
   - les radicaux R₂₇ hydrocarbonés en C₁-C₂₂, linéaires ou ramifiés, saturés ou insaturés, et
   - l'atome d'hydrogène,
R₂₅ est choisi parmi :
   - le radical 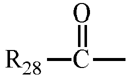
   - les radicaux R₂₉ hydrocarbonés en C₁-C₆, linéaires ou ramifiés, saturés ou insaturés, et
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ; et
X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (I) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 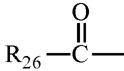
   - les radicaux méthyle, éthyle ou hydrocarbonés en C₁₄-C₂₂, et
   - l'atome d'hydrogène ;
- R₂₅ est choisi parmi :
   - le radical 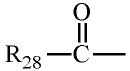 et
   - l'atome d'hydrogène ;
   - R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (I) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthyl ammonium et leurs mélanges.

Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société COGNIS, comme par exemple le produit DEHYQUART F30, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant de 15 à 30 % en poids de méthylsulfate d'acyloxyéthyl-dihydroxyéthyl-méthylammonium, de 45 à 60 % de méthyl sulfate de diacyloxyéthyl-hydroxyéthyl-méthylammonium et de 15 à 30 % de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4,874,554 et US-A-4,137,180.

De préférence, le tensioactif cationique comportant au moins une fonction ester est un sel de diacyloxydialkyl-hydroxyalkylammonium ou un sel de diacyloxytrialkylammonium et en particulier le dipalmitoyléthyl hydroxyéthylammonium méthosulfate, le dicétéaroyléthylhydroxyéthyl méthylammonium méthosulfate, le chlorure de distéaroyléthyldiméthylammonium ou le chlorure de distéaroyldiéthylméthylammonium, et de préférence le dipalmitoyléthyl hydroxyéthylammonium méthosulfate.

Le ou les tensioactifs cationiques comportant au moins une fonction ester utilisés dans la composition selon la présente invention peuvent être présents dans la composition en une teneur allant de 0,1 à 15 % en poids, de préférence de 0,1 à 10 % en poids, mieux de 0,1 à 5 % en poids par rapport au poids total de la composition.

Le rapport pondéral de la quantité de particules de pierre ponce à la quantité de tensioactifs cationiques comportant au moins une fonction ester varie de préférence de 1 à 250, encore plus préférentiellement de 5 à 100, mieux de 10 à 50.

### Polymère non siliconé

La composition capillaire de l'invention comprend en outre un ou plusieurs polymères non siliconés.

Par polymère non siliconé, on entend au sens de la présente invention tout polymère ne comportant pas d'atome de silicium dans sa structure et qui comporte dans ladite structure la répétition d'au moins un motif autre qu'un motif oxyde d'alkylène ou glycérol.

Le ou les polymères non siliconés peuvent être choisis parmi les polymères épaississants. Par polymère épaississant, on entend au sens de la présente invention tout polymère qui par sa présence permet d'augmenter la viscosité de la composition d'au moins 20 centipoises à 25 °C et à un taux de cisaillement de 1s⁻¹. Encore plus préférentiellement on entend par polymère épaississant un polymère qui, introduit à 1 % en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1s⁻¹. Cette viscosité peut être mesurée à l'aide d'un viscosimètre cône/plan (Rhéomètre Haake R600 ou analogue).

Les polymères de l'invention peuvent être non ioniques, anioniques, cationiques ou amphotères.

Plus préférentiellement la composition de l'invention comprend un ou plusieurs polymères épaississants. Encore plus préférentiellement les polymères épaississants de l'invention sont des polymères épaississants de phase aqueuse non ioniques, anioniques, cationiques ou amphotères.

Les polymères épaississants peuvent être des polymères associatifs ou non.

A titre de polymères épaississants selon l'invention il s'agit des polymères épaississants à motifs sucres.

Par motif sucre on entend au sens de la présente invention un motif issu d'un carbohydrate de formule Cₙ(H₂O)ₙ₋₁ ou (CH₂O)ₙ pouvant être éventuellement modifié par substitution et/ou par oxydation et/ou par déshydratation.

Les motifs sucre pouvant entrer dans la composition des polymères épaississants de l'invention sont de préférence issus des sucres suivants : glucose, galactose, arabinose, rhamnose, mannose, xylose, fucose, anhydrogalactose, acide galacturonique, acide glucuronique, acide mannuronique, galactose sulfate anhydrogalactose sulfate.

On peut notamment citer à titre de polymères épaississants de l'invention :
- les gommes natives telles que :
   a) les exsudats d'arbres ou d'arbustes dont :
      - la gomme arabique (polymère ramifié de galactose, d'arabinose, de rhamnose et d'acide glucuronique) ;
      - la gomme ghatti (polymère issu d'arabinose, de galactose, de mannose, de xylose et d'acide glucuronique) ;
      - la gomme karaya (polymère issu d'acide galacturonique, de galactose, de rhamnose et d'acide glucuronique) ; et
      - la gomme tragacanthe (ou adragante) (polymère d'acide galacturonique, de galactose, de fucose, de xylose et d'arabinose).
   b) les gommes issues d'algues dont :
      - l'agar (polymère issu de galactose et d'anhydrogalactose) ;
      - les alginates (polymères d'acide mannuronique et d'acide glucuronique) ; et
      - les carraghénanes et les furcelleranes (polymères de galactose sulfate et d'anhydrogalactose sulfate).
   c) les gommes issus de semences ou tubercules dont :
      - la gomme de guar (polymère de mannose et de galactose) ;
      - la gomme de caroube (polymère de mannose et de galactose) ;
      - la gomme de fenugrec (polymère de mannose et de galactose) ;
      - la gomme de tamarin (polymère de galactose, de xylose et de glucose) ;
      - la gomme de konjac (polymère de glucose et mannose) ;
   d) les gommes microbiennes dont :
      - la gomme de xanthane (polymère de glucose, de mannose acétate, de mannose/acide pyruvique et d'acide glucuronique) ;
      - la gomme de gellane (polymère de glucose partiellement acylé, de rhamnose et d'acide glucuronique) ; et
      - la gomme de scléroglucane (polymère du glucose) ;
   e) les extraits de plantes dont :
      - la cellulose (polymère du glucose) ; et
      - l'amidon (polymère du glucose).

Ces polymères peuvent être modifiés par voie physique ou chimique. A titre de traitement physique on peut citer notamment la température.

A titre de traitements chimiques on peut citer les réactions d'estérification, d'étherification, d'amidification, d'oxydation. Ces traitements permettent de conduire à des polymères qui peuvent être notamment non ioniques, anioniques ou amphotères.

De préférence ces traitements chimiques ou physiques sont appliqués sur les gommes de guar, les gommes de caroube, les amidons et les celluloses.

Les gommes de guar non-ioniques utilisables selon l'invention peuvent être modifiées par des groupements hydroxylakyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.

Les molécules d'amidons utilisées dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons peuvent être modifiés par voie chimique ou physique : notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C₁-C₆ (acétyl), hydroxyalkylés en C₁-C₆ (hydroxyéthyl, hydroxypropyl), carboxyméthyl, octénylsuccinique.

On peut notamment obtenir par réticulation avec des composés phosphorés des phosphates de monoamidon (du type Am-O-PO-(OX)₂), des phosphates de diamidon (du type Am-O-PO-(OX)-O-Am) ou même de triamidon (du type Am-O-PO- (O-Am)₂) ou leurs mélanges (Am signifiant amidon).

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification par voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents, de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 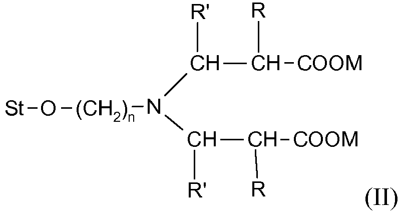 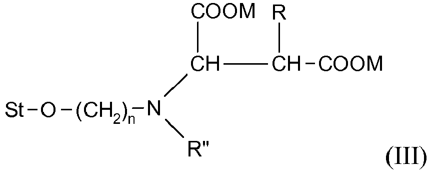 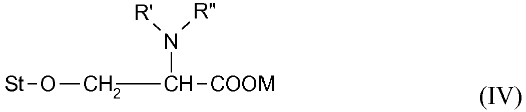 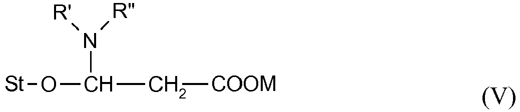 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (III) ou (IV). On utilise plus particulièrement les amidons modifiés par de l'acide 2-chloroéthyl aminodipropionique, c'est à dire les amidons de formule (III) ou (IV) dans lesquelles R, R', R" et M représentent un atome d'hydrogène et n est égal à 2. L'amidon amphotère préféré est un chloroéthylamidodipropionate d'amidon.

Comme indiqué précédemment, les dérivés de celluloses peuvent être notamment anioniques, amphotères ou non-ioniques.

Parmi ces dérivés, on distingue les éthers de celluloses, les esters de celluloses et les esters éthers de celluloses.

Parmi les esters de celluloses, on trouve les esters inorganiques de cellulose par exemple les nitrates, sulfates ou phosphates de cellulose, les esters organiques de cellulose par exemple les monoacétates, triacétates, amidopropionates, acétatebutyrates, acétatepropionates ou acétatetrimellitates de cellulose et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques, on peut citer les alkylcelluloses telles que les méthylcelluloses et les éthylcelluloses (par exemple Ethocel standard 100 Premium de DOW CHEMICAL) ; les hydroxyalkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON) ; les celluloses mixtes hydroxyalkyl-alkylcelluloses telles que les hydroxypropyl-méthylcelluloses (par exemple Methocel E4M de DOW CHEMICAL), les hydroxyéthyl-méthylcelluloses, les hydroxyéthyl-éthylcellulose (par exemple Bermocoll E 481 FQ d'AKZO NOBEL) et les hydroxybutyl-méthylcelluloses.

Parmi les éthers de cellulose anioniques, on peut citer les carboxyalkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les agents épaississants, on peut aussi citer les systèmes épaississants à base de polymères associatifs bien connus de l'homme de l'art et notamment de nature non ionique, anionique, cationique ou amphotère.

Il est rappelé que les polymères associatifs sont des polymères capables, dans un milieu aqueux, de s'associer réversiblement entre eux ou avec d'autres molécules.

Leur structure chimique comprend plus particulièrement au moins une zone hydrophile et au moins une zone hydrophobe.

Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Parmi les polymères associatifs de type cationique, on peut citer :
- (III) les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) vendus par la société AMERCHOL, les produits CRODACEL QM, CRODACEL QL (alkyle en C₁₂) et CRODACEL QS (alkyle en C₁₈) vendus par la société CRODA et le produit SOFTCAT SL 100 vendu par la société AMERCHOL.

Les polymères associatifs de type non ionique utilisables selon l'invention sont choisis de préférence parmi :
- (f) les celluloses ou leurs dérivés, modifiés par des groupements comportant au moins une chaîne grasse tels que des groupes alkyls, arylalkyls, alkylaryls ou leurs mélanges où les groupes alkyls sont en C₈ et en particulier :
   - les alkylhydroxyéthylcelluloses non-ioniques telles que les produits NATROSOL PLUS GRADE 330 CS et POLYSURF 67 (alkyle en C₁₆) vendus par la société AQUALON ;
   - les nonoxynylhydroxyéthylcelluloses non-ioniques telles que le produit AMERCELL HM-1500 vendu par la société AMERCHOL ;
   - les alkylcelluloses non-ioniques telles que le produit BERMOCOLL EHM 100 vendu par la société BEROL NOBEL ;
- (g) les dérivés de guar associatifs comme les hydroxypropylguars modifiés par une chaîne grasse tel que le produit ESAFLOR HM 22 (modifié par une chaîne alkyle en C₂₂) vendu par la société LAMBERTI ; le produit MIRACARE XC 95-3 (modifié par une chaîne alkyle en C₁₄) et le produit RE 205-146 (modifié par une chaîne alkyle en C₂₀) vendus par RHODIA CHIMIE.

Tout particulièrement les polymères non siliconés de l'invention sont choisis parmi les polymères à motifs sucre associatifs ou non, mieux parmi les polysaccharides de type hydroxyéthylcellulose et hydroxypropyl guar.

Le ou les polymères non siliconés peuvent notamment être présents en une teneur allant de 0,01 à 20 % en poids, de préférence de 0,1 à 10 % en poids, mieux de 0,2 à 5 % en poids, par rapport au poids total de la composition.

Le rapport pondéral de la quantité de particules de pierre ponce à la quantité de polymères non siliconés varie de préférence de 1 à 200, encore plus préférentiellement de 3 à 100, mieux de 5 à 50.

La composition capillaire selon l'invention peut également comprendre en outre au moins une silicone à groupements ammonium quaternaire.

Conformément à l'invention, on entend par « silicone à groupements ammonium quaternaire » toute silicone comportant un ou plusieurs groupements ammonium quaternaire. Ces groupements ammonium quaternaire peuvent être liés en position alpha ou oméga ou sous forme de groupements latéraux. Ils peuvent être liés directement au squelette polysiloxane ou peuvent être portés par des chaînes hydrocarbonées.

Selon l'invention, on entend par silicone, en conformité avec l'acceptation générale, tout polymère ayant une structure basée sur l'alternance d'atomes de silicium et d'oxygène, reliés entre eux par des liaisons dites liaisons siloxane (-Si-O-Si-), et caractérisée en outre par l'existence de liaisons silicium-carbone. Ces silicones, ou polysiloxanes, sont généralement obtenues par polycondensation de silanes convenablement fonctionnalisés. Les radicaux hydrocarbonés les plus courants portés par les atomes de silicium sont les radicaux alkyle inférieurs, en particulier méthyle, les radicaux fluoroalkyles, les radicaux aryle et en particulier phényle.

Les silicones à groupements ammonium quaternaire de la présente invention sont par exemple choisies parmi les composés correspondants aux formules générales suivantes : 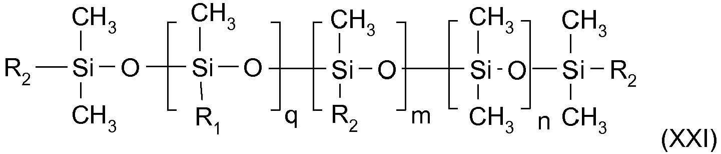 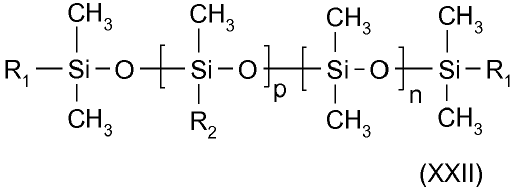 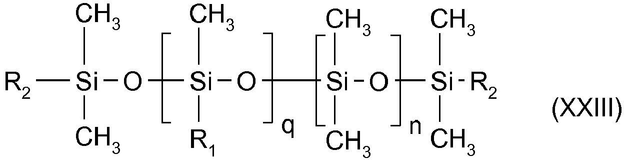 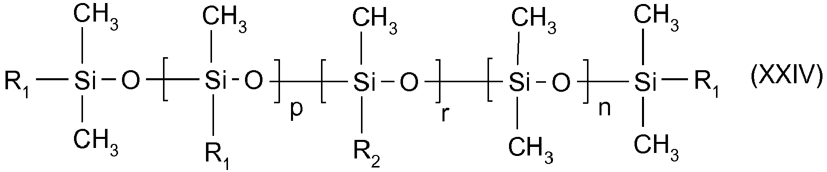 dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-(PO₃H)_{d}-R₅ ou -C_{c}H_{2c}-O-(C₄H₈O)ₐ-(PO₃H)_{d}-R₅ ;
   R₅, identique ou différent, est choisi parmi les groupes de formule suivante : 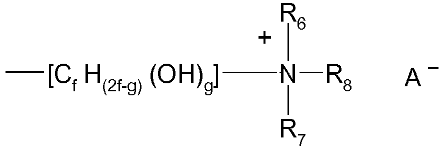
- les radicaux R₈ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZCOR₉; et
- R₆, R₇ et R₉, identiques ou différents, représentent des radicaux alkyle en C₁₋₂₂ ou alcényle en C_{2-22'}, linéaires ou ramifiés, portant éventuellement un ou plusieurs groupements OH, ou R₇ peut former avec une partie de R₈ un hétérocycle (cycle avec au moins un hétéroatome tel que par exemple N, O, P), l'hétérocycle est notamment une imidazoline.

De préférence R₆ et R₇ désignent un radical alkyle en C₁-C₆ et plus particulièrement méthyle, R₉ désigne de préférence un radical choisi parmi les alkyle en C₈-C₁₈ et les alcényle en C₈-C₁₈ et notamment un radical cocoyle.
- m varie de 0 à 20 ;
- n varie de 0 à 500 ;
- p varie de 1 à 50 ;
- q varie de 0 à 20 ;
- r varie de 1 à 20 ;
- a varie de 0 à 50 ;
- b varie de 0 à 50 ;
- c varie de 0 à 4 ;
- d désigne 0 ou 1
- f varie de 0 à 4,
- g varie de 0 à 2, de préférence est égal à 1 ; et
- h varie de 1 à 4, de préférence est égal à 3 ;
Z représente un atome d'oxygène ou NH,
A⁻ représente un anion minéral ou organique monovalent tel qu'un halogénure (par exemple chlorure, bromure), un sulfate, ou un carboxylate (par ex. acétate, lactate, citrate).

De préférence on utilise les silicones à groupements ammonium quaternaire de formule (XXII) ou (XXIII).

De préférence, on utilise les silicones à groupements ammonium quaternaire répondant à la formule générale (XXIII) telle que définie ci-dessus, et plus particulièrement celles répondant à la formule générale (XXIII) dans laquelle au moins l'une des, et de préférence toutes les conditions suivantes sont satisfaites :
- c est égal à 0;
- d désigne 0 ;
- a est égal à zéro ;
- b est égal à 1
- n varie de 0 à 100 ;
- q est égal à 0 ;
- f = 3 ;
- g = 1 ;
- R₆ et R₇ désignent le groupe méthyle ; et
- R₈ désigne un radical alkyle C₁₀-C₂₂.

Parmi les silicones de l'invention, on peut citer par exemple celles commercialisées par la société GOLDSCHMIDT sous les dénominations ABIL QUAT 3272, ABIL B 9905, ABIL QUAT 3474 et ABIL K 3270, par la société LIPO FRANCE sous les dénominations SILQUAT Q-100, SILQUAT Q-200 WS, SILQUAT AX, SILQUAT AC, SILQUAT AD et SILQUAT AM tous fabriqués par la société SILTECH, par la société OSI sous la dénomination MAGNASOFT EXHAUST et SILSOFT C-880 et par la société UCIB sous les dénominations PECOSIL 14-PQ et PECOSIL 36-PQ (fabricant PHOENIX CHEMICAL). Ces silicones sont notamment décrites dans les brevets EP 0 530 974, DE 3 719 086, DE 3 705 121, EP 0 617 607 et EP 0 714 654.

Selon un mode de réalisation, la silicone à groupements ammonium quaternaire est de formule (XXIII). Encore plus préférentiellement la silicone à groupements ammonium quaternaire est le composé référencé au CTFA (nom INCI) sous l'appellation Quaternium-80.

Les silicones à groupements ammonium quaternaire utilisées conformément à l'invention peuvent se présenter sous forme de solutions aqueuses ou éventuellement sous forme de dispersions ou d'émulsions dans l'eau.

La composition capillaire peut comporter des silicones à groupements ammonium quaternaire en une teneur allant de 0,1 à 20 % en poids, de préférence de 0,2 à 10 % en poids, mieux de 0,3 à 5 % en poids par rapport au poids total de la composition.

Le rapport pondéral de la quantité de particules de pierre ponce à la quantité de silicones à groupements ammonium quaternaire varie de préférence de 1 à 250, encore plus préférentiellement de 5 à 200, mieux de 10 à 100.

### Autres composants de la composition capillaire

Outre les particules de pierre ponce, le ou les tensioactifs cationiques comportant au moins une fonction ester et le ou les polymères non siliconés conformes à l'invention, la composition capillaire peut comprendre un ou plusieurs additifs.

La composition capillaire peut ainsi comprendre un ou plusieurs tensioactifs additionnels tels que des tensioactifs anioniques, amphotères, zwittérioniques ou non ioniques.

Le ou les tensioactifs additionnels sont de préférence choisis parmi les tensioactifs non ioniques.

Les agents tensioactifs non-ioniques sont des composés bien connus en soi (voir notamment à cet égard « Handbook of Surfactants » par M.R. Porter, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Le ou les tensioactifs non ioniques peuvent être présents dans la composition capillaire dans des concentrations allant de 0,1 à 25 % en poids, de préférence de 1 à 20 % en poids par rapport au poids total de la composition.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits vendus sous la dénomination MIRANOL, tel que décrits dans les brevets US 2,528,378 et US 2,781,354 et classés dans le dictionnaire CTFA, 3ème édition, 1982, sous les dénominations amphocarboxyglycinates et amphocarboxypropionates de structures respectives :

R₂ -CONHCH₂CH₂-N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂' -CONHCH₂CH₂-N(B)(C)

dans laquelle :
B représente -CH₂CH₂OX', C représente -(CH₂)_{Z}-Y', avec z = 1 ou 2,
X' désigne le groupement -CH₂CH₂-COOH ou un atome d'hydrogène,
Y' désigne -COOH ou le radical -CH₂CHOH-SO₃H, et
R₂' désigne un radical alkyle d'un acide R₉-COOH présent dans l'huile de coprah ou dans l'huile de lin hydrolysée, un radical alkyle, notamment en C₇, C₉, C₁₁, ou C₁₃, un radical alkyle en C₁₇ et sa forme iso, un radical C₁₇ insaturé.

Ces composés sont classés dans le dictionnaire CTFA, 5ème édition, 1993, sous les dénominations disodium cocoamphodiacetate, disodium lauroamphodiacetate, disodium caprylamphodiacetate, disodium capryloamphodiacetate, disodium ocoamphodipropionate, disodium lauroamphodipropionate, disodium caprylampho-dipropionate, disodium capryloamphodipropionate, lauroamphodipropionic acid, cocoamphodipropionic acid, disodium cocoamphocarboxyl ethyl hydroxypropyl sulfonate.

A titre d'exemple, on peut citer le cocoamphodiacetate commercialisé sous la dénomination commerciale MIRANOL^{®} C2M concentré par la société Rhodia Chimie.

Pour ce qui concerne les tensioactifs anioniques, on peut citer de manière non limitative les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucosides citrates, les alkylpolyglycosides tartrates et les alkylpolyglycosides sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les tensioactifs anioniques éventuellement présents sont de préférence des tensioactifs anioniques doux.

En ce qui concerne les tensioactifs anioniques doux, on peut citer notamment les composés suivants et leurs sels, ainsi que leurs mélanges :
- les acides alkyl éther carboxyliques polyoxyalkylénés ;
- les acides alkylaryl éther carboxyliques polyoxyalkylénés ;
- les acides alkylamido éther carboxyliques polyoxyalkylénés en particulier ceux comportant 2 à 50 groupements oxyde d'éthylène ;
- les acides d'alkyl D galactoside uroniques ;
- les acylsarcosinates, les acylglutamates ; et
- les esters d'alkylpolyglycosides carboxyliques.

Tout particulièrement, on peut utiliser des acides alkyl éther carboxyliques polyoxyalkylénés comme par exemple l'acide lauryl ether carboxylique (4,5 OE) commercialisé par exemple sous la dénomination AKYPO RLM 45 CA de KAO.

Si de tels tensioactifs anioniques ou amphotères sont présents, alors leur teneur va de 0,1 à 20 % en poids, par rapport au poids total de la composition capillaire, plus particulièrement de 1 à 10 % en poids, par rapport au poids total de la composition.

De préférence, la composition capillaire ne contient pas de tensioactif détergent anionique de type sulfate (alkyl sulfate ou alkyl éther sulfate, alkyl amido éther sulfate). Et si elle en contient, sa teneur est telle que le rapport pondéré : tensioactif détergent anionique de type alkyl sulfate ou alkyl éther sulfate/somme des autres tensioactifs non cationiques, soit de préférence inférieur ou égal à 1, et plus particulièrement inférieur ou égal à 0,75 et encore plus préférentiellement inférieur ou égal à 0,5.

La composition capillaire peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les parfums, les peptisants, les conservateurs, les vitamines, les agents antipelliculaires, les agents antiséborrhéïques, les agents antichute des cheveux, les agents épaississants non polymériques tels que les amides grasses, les éthers gras, les alcools gras, les silices, les argiles, etc.

Les adjuvants cités ci-dessus sont en général présents en quantité comprise, pour chacun d'eux, entre 0,01 et 20 % en poids par rapport au poids de la composition.

La composition capillaire peut comprendre un ou plusieurs agents conditionneurs non polymériques ou siliconés non quaternaires additionnels.

Lorsque la composition capillaire contient au moins un agent conditionneur additionnel, celui-ci peut être choisi parmi les huiles de synthèse telles que les poly-α-oléfines, les huiles fluorées, les cires fluorées, les gommes fluorées, les esters d'acides carboxyliques, des tensioactifs cationiques distincts de ceux requis selon l'invention, les silicones non quaternaires, les huiles minérales, végétales ou animales, les céramides, les pseudocéramides, et leurs mélanges.

Parmi les tensioactifs cationiques distincts de ceux requis selon l'invention, on peut citer en particulier (liste non limitative) : les sels d'amines grasses primaires, secondaires ou tertiaires, éventuellement polyoxyalkylénées ; les dérivés d'imidazoline ; les sels d'ammonium quaternaire et en particulier les sels de trialkylammonium comportant au moins une chaîne grasse ayant de 10 à 30 atomes de carbone et notamment les sels de cétyltriméthymammonium ou de béhényltriméthylammonium.

La teneur en agents conditionneurs additionnels non polymériques ou siliconés non quaternaires dans la composition capillaire peut aller de 0,001 % à 10 % en poids, de préférence de 0,005 % à 5 % en poids, et encore plus préférentiellement de 0,01 % à 3 % en poids par rapport au poids total de la composition finale.

Le milieu aqueux acceptable pour les cheveux peut comporter de l'eau ou un mélange d'eau et un ou plusieurs solvants organiques acceptables sur le plan cosmétique.

La teneur en eau de la composition est de préférence supérieure ou égale à 50 % en poids par rapport au poids total de la composition.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Le ou les solvants organiques peuvent être présents dans des proportions par exemple comprises entre 1 et 40 % en poids environ par rapport au poids total de la composition capillaire, et encore plus préférentiellement entre 5 et 30 % en poids environ.

### Forme galénique

La composition capillaire selon l'invention peut se présenter sous différentes formes galéniques telles qu'une lotion, un shampoing, un gel, une crème, une cire. La composition capillaire peut être conditionnée dans tout type de récipient avec applicateur ou non. Le récipient peut contenir une bille ou un organe permettant notamment d'homogénéiser la composition capillaire avant application de celle-ci sur les cheveux. Le récipient contenant la composition capillaire peut avoir une contenance supérieure ou égale à 15 ml, notamment supérieure ou égale à 50 ml, voire à 100 ml, notamment supérieure ou égale à 150 ml, par exemple comprise entre 15 ml et 500 ml.

La composition capillaire peut imprégner une lingette.

La composition capillaire peut être introduite dans un courant de fluide vecteur, et être projetée sur les cheveux.

Dans un mode de réalisation particulier, la composition capillaire peut être contenue dans un réservoir d'un peigne ou d'une brosse présentant par exemple des orifices à la base des dents ou poils permettant d'amener le produit et de l'appliquer sur les cheveux.

### Procédé de traitement

L'invention concerne également un procédé de traitement des cheveux consistant à appliquer sur les cheveux au moins une composition capillaire telle que définie précédemment, puis éventuellement à effectuer un rinçage.

Le traitement, par exemple l'abrasion, des cheveux peut avoir lieu immédiatement après l'application de la composition capillaire sur les cheveux, par exemple dans un délai inférieur à une heure, notamment inférieur à 30 min, voire à 10 min après l'application de la composition capillaire sur les cheveux. Un rinçage des cheveux, lorsqu'il a lieu, peut intervenir rapidement après traitement, par exemple abrasion, des cheveux. Le temps entre l'application de la composition et le rinçage peut par exemple être inférieur à 4h, notamment inférieur à 1h, par exemple inférieur à 20 min.

L'étape de traitement, par exemple d'abrasion, des cheveux peut être effectuée en frottant les cheveux avec les mains nues ou en interposant au moins une surface entre les mains et les cheveux chargés de composition capillaire, par exemple une lingette ou serviette, un gant ou autre. Le traitement peut être effectué autrement qu'à l'aide d'un peigne ou d'une brosse.

Le traitement, par exemple l'abrasion, des cheveux peut par exemple être réalisé à l'aide d'au moins une surface mise en mouvement, en vibration ou en rotation notamment, par un système mécanisé.

Le traitement à l'aide de la composition capillaire peut par exemple être effectué, à la main par exemple, mèche par mèche, par exemple en deux mouvements : un premier mouvement dit « tangentiel », c'est-à-dire sensiblement le long des cheveux, par exemple de la racine vers la pointe, et un deuxième mouvement dit « de cisaillement », c'est-à-dire sensiblement orthogonalement aux cheveux, dans une direction transversale à ceux-ci. Les deux mouvements peuvent être répétés plusieurs fois, par exemple au moins cinq fois, par exemple dix fois. En variante, un seul des mouvements précités peut être effectué et éventuellement répété plusieurs fois.

La durée de traitement, par exemple d'abrasion, au moyen de la composition conforme à l'invention peut dépendre par exemple de l'intensité recherchée de l'abrasion et de l'état du cheveu.

Le procédé selon l'invention peut comporter deux étapes de traitement, par exemple d'abrasion, à l'aide de deux compositions capillaires successivement appliquées, chacune comportant des particules de pierre ponce conformes à l'invention, les diamètres moyens en volume ou la dureté des particules de pierre ponce conformes à l'invention des deux compositions capillaires étant différentes.

Un flacon unique peut contenir les deux compositions capillaires conditionnées séparément, ou deux flacons différents peuvent contenir chacun une composition capillaire.

Un autre mode de conditionnement peut consister en un flacon contenant une composition capillaire de base dépourvue de particules de pierre ponce conformes à l'invention, lesquelles sont conditionnées à part, par exemple dans au moins deux compartiments séparés, en fonction de leur granulométrie ou de leur dureté. Dans ce dernier cas, l'utilisateur choisit avant le traitement, par exemple l'abrasion, les particules de pierre ponce conformes à l'invention à mélanger à la composition de base pour former la première composition capillaire, le mélange étant effectué par l'utilisateur ou au sein du flacon. L'utilisateur peut, après le premier traitement, par exemple la première abrasion, effectuer un deuxième traitement, par exemple une deuxième abrasion, en mélangeant cette fois-ci d'autres particules de pierre ponce conformes à l'invention à la composition capillaire de base pour former la deuxième composition capillaire. Un rinçage peut être effectué entre et/ou après les deux traitements.

Le procédé de traitement peut être mis en oeuvre après une étape de caractérisation du cheveu, par exemple à l'aide d'un examen visuel à l'oeil nu ou sous un dispositif d'agrandissement ou par voie instrumentale, par exemple en enregistrant le son produit par le déplacement d'un peigne dans les cheveux à l'aide d'un sonomètre ou en déterminant optiquement la brillance des cheveux. La caractérisation du cheveu peut encore faire intervenir un réactif chimique appliqué sur un échantillon de cheveu.

Le procédé selon l'invention peut comporter l'étape consistant à peigner et/ou rincer les cheveux après application de la composition capillaire. Le rinçage peut être effectué à l'eau.

Le procédé selon l'invention peut également comporter l'étape consistant à chauffer les cheveux avant ou après mise en contact avec la composition capillaire, par exemple à une température comprise entre 40 °C et 250 °C, notamment entre 60 °C et 220 °C. On peut par exemple chauffer les cheveux après le traitement, par exemple l'abrasion, de manière à les mettre en forme, par exemple en effectuant un brushing. Le chauffage des cheveux peut par exemple être effectué au moyen d'un fer, d'un mélange eau liquide/vapeur d'eau ou au moyen d'un casque chauffant.

Les cheveux peuvent être séchés totalement ou partiellement.

### Application d'un produit de traitement

Le procédé selon l'invention peut comporter en outre l'étape consistant à appliquer, par exemple avant ou après traitement à l'aide de la composition capillaire selon l'invention, un autre produit de traitement sur les cheveux. Le produit de traitement peut être par exemple un produit cosmétique, notamment un conditionneur, une permanente, un défrisant, un produit de décoloration ou de coloration des cheveux.

Le produit de traitement peut être par exemple choisi parmi les suivants, cette liste n'étant pas limitative:
- les produits visant à modifier les propriétés mécaniques des cheveux, notamment comportant un réducteur, tel que l'acide thioglycolique et ses dérivés, la cystéine, le sulfite, la soude, le carbonate de guanidine, la trihydroxyméthyl phosphine, ou un oxydant, tel que H₂O₂, le persulfate ;
- les produits émollients ou de pénétration, comportant par exemple un solvant, un glycol, un plastifiant, ou un tensioactif cationique, anionique ou amphotère ;
- les produits modifiant les propriétés de la surface du cheveu, comportant notamment une silicone, une silicone aminée réactive, un polymère adhésif, un lubrifiant non siliconé comportant des corps gras choisis parmi les huiles végétales, les huiles minérales, les huiles de synthèse, les cires notamment des alcools gras ou des esters gras ;
- les produits restructurant l'intérieur du cheveu comportant par exemple un ionène, une protéine, un hydroxyacide ou un composé réactif, notamment un générateur de formol, un silane ; et
- les colorants directs ou d'oxydation.

Le produit de traitement peut être appliqué préalablement au traitement, par exemple de type abrasion, et peut contribuer à protéger les cheveux pendant celui-ci, afin d'éviter notamment une abrasion excessive.

Le produit de traitement est de préférence appliqué après application de la composition conforme à l'invention.

Ainsi, selon un mode de réalisation, le procédé conforme à l'invention peut comporter en outre l'étape consistant à soumettre les cheveux à un post-traitement, après traitement à l'aide d'une composition capillaire telle que définie précédemment, le post-traitement étant choisi parmi l'application d'un conditionneur, d'une permanente, d'un défrisant, d'un produit de coloration ou de décoloration des cheveux.

### Kits de traitement

L'invention a encore pour objet, indépendamment ou en combinaison avec ce qui précède, un kit de traitement des cheveux comportant :
- une composition capillaire comprenant au moins des particules de pierre ponce, un ou plusieurs tensioactifs cationiques comportant au moins une fonction ester et un ou plusieurs polymères non siliconés et
- un support comportant des instructions pour l'emploi de la composition capillaire sur les cheveux, par exemple en vue de réaliser une abrasion des cheveux.

La composition capillaire est telle que décrite plus haut.

Le kit peut comporter en outre une composition de post-traitement des cheveux. La composition de post-traitement peut être choisie parmi un conditionneur, une permanente, un défrisant, un produit de coloration ou de décoloration des cheveux. Le produit de post-traitement peut par exemple être choisi parmi ceux décrits plus haut.

### EXEMPLE :

(les proportions sont pondérales par rapport au poids total de la composition)

### Exemple comparatif 1 - Mise en évidence du pouvoir lissant

Les compositions de traitement suivantes ont été élaborées :

| Composition | A | B |
|---|---|---|
| Caprylyl glycol | 0,5 | 0,5 |
| Cétrimonium chloride | 0,5 | 0,5 |
| Pumice (Pierre Ponce 0 ½ D d'EYRAUD) | 18 | 0 |
| Quaternium-80 (ABIL QUAT 3272 de GOLDSCHMIDT) | 0,2 | 0,2 |
| Glycéryl stéarate | 1,2 | 1,2 |
| Hydroxypropyl guar (JAGUAR HP 105 de RHODIA) | 0,1 | 0,1 |
| Glycérine | 2 | 2 |
| Propylène glycol | 0,5 | 0,5 |
| Cétéaryl alcohol | 4 | 4 |
| Hydroxyéthylcellulose (NATROSOL 250 HHR de AQUALON) | 0,8 | 0,8 |
| Conservateurs | 0,2 | 0,2 |
| Dipalmitoyléthyl hydroxyéthylmonium méthosulfate/Cétéaryl alcohol (30/70 en poids) (DEHYQUART F30 de COGNIS) | 4,3 | 4,3 |
| Eau | Qsp 100 | Qsp 100 |

L'étude est réalisée sur un panel de 20 femmes : 10 femmes aux cheveux longs naturels et 10 femmes aux cheveux longs sensibilisés.

10 à 15 g de chacun de ces traitements sont appliqués par demi-tête.

L'application se fait mèche par mèche en deux mouvements : un premier mouvement dit « tangentiel » et un deuxième mouvement dit « de cisaillement ». Les deux mouvements sont répétés 10 fois. Les cheveux sont peignés, puis rincés et enfin séchés par brushing.

### Analyse sensorielle

Les résultats montrent que pour 7 femmes aux cheveux naturels sur 10 et pour 9 femmes aux cheveux sensibilisés sur 10, le côté traité par le procédé de l'invention avec la composition A est plus lisse, plus souple et plus homogène que le côté traité avec la composition B. L'invention permet de réduire la masse et de maîtriser le volume des cheveux et d'améliorer la discipline de la chevelure. Les effets sont d'autant plus remarquables que les cheveux sont sensibilisés et épais.

Après 5 shampooings, le lissage des cheveux est conservé ainsi que la maîtrise du volume quel que soit le degré de sensibilité des cheveux (naturels à très sensibilisés).

### Exemple 2 :

La composition suivante a été préparée :

| | |
|---|---|
| Caprylyl glycol | 0,5 |
| Cétrimonium chloride | 0,5 |
| Pumice (0-D Ponce décontaminée d'EYRAUD de taille inférieure à 125 µm) | 25 |
| Quaternium-80 (ABIL QUAT 3272 de GOLDSCHMIDT) | 0,2 |
| Glycéryl stéarate | 1,2 |
| Hydroxypropyl guar (JAGUAR HP 105 de RHODIA) | 0,1 |
| Glycérine | 2 |
| Propylène glycol | 0,5 |
| Cétéaryl alcohol | 1 |
| Hydroxyéthylcellulose (NATROSOL 250 HHR de AQUALON) | 0,8 |
| Conservateurs | 0,2 |
| Dipalmitoyléthyl hydroxyéthylmonium méthosulfate /Cétéaryl alcohol (30/70 en poids) (DEHYQUART F30 de COGNIS) | 4,3 |
| Eau | Qsp 100 |

### Exemple 3 :

La composition suivante a été préparée :

| | |
|---|---|
| Caprylyl glycol | 0,1 |
| Cétrimonium chloride | 0,5 |
| Pumice (Pierre Ponce 0 ½ D d'EYRAUD) | 15 |
| Glycéryl stéarate | 1,2 |
| Hydroxypropyl guar (JAGUAR HP 105 de RHODIA) | 0,1 |
| Glycérine | 2 |
| Propylène glycol | 0,5 |
| Cétéaryl alcohol | 1 |
| Hydroxyéthylcellulose (NATROSOL 250 HHR de AQUALON) | 0,8 |
| Conservateurs | 0,2 |
| Dipalmitoyléthyl hydroxyéthylmonium méthosulfate /Cétéaryl alcohol (30/70 en poids) (DEHYQUART F30 de COGNIS) | 4,3 |
| Eau | Qsp 100 |

L'invention n'est pas limitée aux exemples décrits.

L'expression « comportant un » doit être comprise comme étant synonyme de « comportant au moins un » et « compris entre » ou « allant de » s'entendent bornes incluses, sauf si le contraire est spécifié.

## Revendications

1. Composition capillaire comprenant au moins des particules de pierre ponce, un ou plusieurs tensioactifs cationiques comportant au moins une fonction ester et un ou plusieurs polymères non siliconés, lesdits polymères non siliconées étant choisis parmi les polymères épaississants à motifs sucres, et ledit tensioactif cationique comportant au moins une fonction ester étant choisi parmi les sels d'ammonium quaternaire contenant au moins une fonction ester de formule (I) suivante : 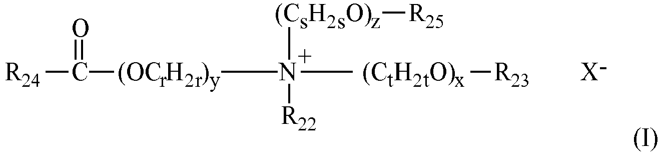 dans laquelle :
R₂₂ est choisi parmi les radicaux alkyles en C₁-C₆ et les radicaux hydroxyalkyles ou dihydroxyalkyles en C₁-C₆;
R₂₃ est choisi parmi :
- le radical 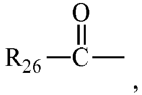
- les radicaux R₂₇ hydrocarbonés en C₁-C₂₂, linéaires ou ramifiés, saturés ou insaturés, et
- l'atome d'hydrogène,
R₂₅ est choisi parmi :
- le radical 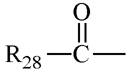
- les radicaux R₂₉ hydrocarbonés en C₁-C₆, linéaires ou ramifiés, saturés ou insaturés, et
- l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ; et
X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

2. Composition selon la revendication 1, les particules de pierre ponce ayant un diamètre moyen en volume inférieur ou égal à 500 µm, de préférence compris entre 50 et 500 µm, mieux inférieur ou égal à 300 µm, par exemple compris entre 100 et 300 µm.

3. Composition selon l'une des revendications précédentes, dans laquelle les particules de pierre ponce sont présentes en une teneur allant de 0,1 % à 35 % en poids, notamment de 5 % à 30 % en poids, en particulier de 10 % à 25 % en poids, par exemple de 15 % à 20 % en poids, mieux de 18 % à 20 % en poids par rapport au poids total de la composition.

4. Composition selon l'une quelconque des revendications précédentes, dans laquelle les particules de pierre ponce ont une dureté allant de 3 à 10 Mohs, voire supérieure ou égale à 4, par exemple supérieure ou égale à 5, et en particulier allant de 5 à 5,5 sur l'échelle de Mohs.

5. Composition selon l'un quelconque des revendications précédentes, dans laquelle :
R₂₂ désigne un radical méthyle ou éthyle,
x et y sont égaux à 1,
z est égal à 0 ou 1,
r, s et t sont égaux à 2,
R₂₃ est choisi parmi :
- le radical 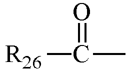
les radicaux méthyle, éthyle ou hydrocarbonés en C₁₄-C₂₂,
l'atome d'hydrogène ;
R₂₅ est choisi parmi :
- le radical 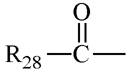 et
- l'atome d'hydrogène ;
R₂₄, R₂₆ et R₂₈, identiques ou différents, étant choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

6. Composition selon l'une quelconque des revendications précédentes, dans laquelle le tensioactif cationique comportant au moins une fonction ester est un sel de diacyloxydialkyl-hydroxyalkylammonium ou un sel de diacyloxytrialkylammonium et en particulier le dipalmitoyléthyl hydroxyéthylammonium méthosulfate, le dicétéaroyléthylhydroxyéthyl méthylammonium méthosulfate, le chlorure de distéaroyléthyldiméthylammonium ou le chlorure de distéaroyldiéthylméthylammonium

7. Composition selon l'une quelconque des revendications précédentes, dans laquelle le ou les tensioactifs cationiques comportant au moins une fonction ester sont présents en une teneur allant de 0,1 à 15 % en poids, de préférence de 0,1 à 10 % en poids, mieux de 0,1 à 5 % en poids, par rapport au poids total de la composition.

8. Composition selon l'une quelconque des revendications précédentes, dans laquelle les polymères non siliconés sont choisis parmi les polymères à motif sucre associatifs ou non, notamment parmi les polysaccharides tels que l'hydroxyéthylcellulose et l'hydroxypropyl guar.

9. Composition selon l'une quelconque des revendications précédentes, dans laquelle le ou les polymères non siliconés sont présents en une teneur allant de 0,01 à 20 % en poids, de préférence de 0,1 à 10 % en poids, mieux de 0,2 à 5 % en poids, par rapport au poids total de la composition.

10. Composition selon l'une quelconque des revendications précédentes, comprenant en outre au moins une silicone à groupements ammonium quaternaire.

11. Composition selon la revendication précédente, **caractérisée en ce que** ladite silicone est le Quaternium-80.

12. Procédé de traitement des cheveux consistant à appliquer sur les cheveux au moins une composition capillaire selon l'une quelconque des revendications précédentes, puis éventuellement à effectuer un rinçage.

13. Procédé selon la revendication précédente, comportant en outre l'étape consistant à soumettre les cheveux à un post-traitement, après traitement à l'aide d'une composition capillaire selon l'une quelconque des revendications 1 à 11, le post-traitement étant choisi parmi l'application d'un conditionneur, d'une permanente, d'un défrisant, d'un produit de coloration ou de décoloration des cheveux.

14. Procédé selon l'une quelconque des revendications 12 et 13, l'étape de traitement des cheveux étant effectuée en frottant les cheveux avec les mains nues ou en interposant au moins une surface entre les mains et les cheveux chargés de composition capillaire.

15. Kit de traitement des cheveux comportant :
- une composition capillaire telle que définie en revendication 1, et
- un support comportant des instructions pour l'emploi de la composition capillaire sur les cheveux.

16. Kit selon la revendication précédente, comportant en outre une composition de post-traitement des cheveux, de préférence choisie parmi un conditionneur, une permanente, un défrisant, un produit de coloration ou de décoloration des cheveux.

## Patentansprüche

1. Haarzusammensetzung umfassend mindestens Bimssteinteilchen, ein oder mehrere kationische Tenside, umfassend mindestens eine Esterfunktion und ein oder mehrere nicht silikonhaltige Polymere, wobei die Nichtsilikonpolymere ausgewählt sind aus verdickenden Polymeren mit Zuckereinheiten und das katonische Tensid mindestens eine Esterfunktion umfasst, die aus quaternären Ammoniumsalzen ausgewählt ist, die mindestens eine Esterfunktion der folgenden Formel (I) enthalten: 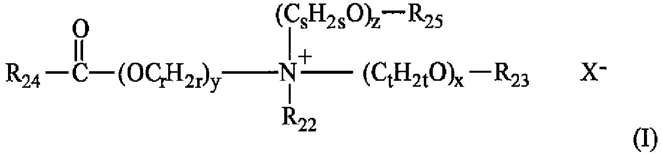 wobei:
R₂₂ ausgewählt ist aus C₁-C₆-Alkylresten und C₁-C₆-Hydroxyalkyl oder C₁-C₆-Dihydroxyalkylresten;
R₂₃ ausgewählt ist aus:
- dem Rest 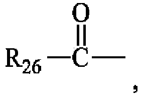
- den linearen oder verzweigten, gesättigten oder ungesättigten C₁-C₂₂-Kohlenwasserstoffresten R₂₇, und
- dem Wasserstoffatom,
R₂₅ ausgewählt ist aus:
- dem Rest 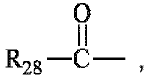
- den linearen oder verzweigten, gesättigten oder ungesättigten C₁-C₆-Kohlenwasserstoffresten R₂₉, und
- dem Wasserstoffatom,
R₂₄, R₂₆ und R₂₈, die gleich oder verschieden sind, aus linearen oder verzweigten, gesättigten oder ungesättigten C₇-C₂₁-Kohlenwasserstoffresten ausgewählt sind;
r, s und t, die gleich oder verschieden sind, ganze Zahlen von 2 bis 6 sind;
y eine ganze Zahl von 1 bis 10 ist;
x und z, die gleich oder verschieden sind, ganze Zahlen von 0 bis 10 sind; und
X⁻ ein einfaches oder komplexes, organisches oder anorganisches Anion ist;
mit der Maßgabe, dass die Summe x+y+z 1 bis 15 beträgt, dass wenn x 0 ist, R₂₃ R₂₇ bezeichnet, und dass wenn z 0 ist, R₂₅ R₂₉ bezeichnet.

2. Zusammensetzung nach Anspruch 1, wobei die Bimssteinteilchen einen volumengemittelten Durchmesser von kleiner oder gleich 500 µm, vorzugsweise zwischen 50 und 500 µm, stärker bevorzugt von kleiner oder gleich 300 µm, beispielsweise zwischen 100 und 300 µm aufweisen.

3. Zusammensetzung nach einem der vorangehenden Ansprüche, wobei die Bimssteinteilchen in einem Gehalt von 0,1 bis 35 Gew.-%, insbesondere von 5 bis 30 Gew.-%, spezieller von 10 bis 25 Gew.-%, beispielsweise von 15 bis 20 Gew.-%, stärker bevorzugt von 18 bis 20 Gew.-% bezogen auf das Gesamtgewicht der Zusammensetzung aufweisen.

4. Zusammensetzung nach einem der vorangehenden Ansprüche, wobei die Bimssteinteilchen eine Härte von 3 bis 10 Mohs, sogar größer oder gleich 4, beispielsweise größer oder gleich 5, und insbesondere von 5 bis 5,5 auf der Mohs-Skala aiufweisen.

5. Zusammensetzung nach einem der vorangehenden Ansprüche, wobei:
R₂₂ einen Methyl- oder Ethylrest bezeichnet,
x und y gleich 1 sind,
z gleich 0 oder 1 ist,
r, s und t gleich 2 sind,
R₂₃ ausgewählt ist aus:
- dem Rest 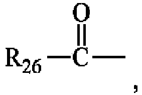
den Methyl-, Ethyl- oder C₁₄-C₂₂-Kohlenwassersoffresten,
dem Wasserstoffatom;
R₂₅ ausgewählt ist aus:
dem Rest 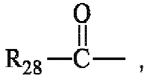
- dem Wasserstoffatom;
R₂₄, R₂₆ und R₂₈, die gleich oder verschieden sind, ausgewählt sind aus linearen oder verzweigten, gesättigten oder ungesättigten C₁₃-C₁₇-Kohlenwasserstoffresten, und vorzugsweise aus linearen oder verzweigten, gesättigten oder ungesättigten C₁₃-C₁₇-Alkyl- oder Alkenylresten.

6. Zusammensetzung nach einem der vorangehenden Ansprüche, wobei das kationsische Tensid, das mindestens eine Esterfunktion umfasst, ein Diacyloxydialkylhydroxyalkylammoniumsalz oder ein Diacyloxytrialkylammoniumsalz und insbesondere Dipalmitoylethylhydroxyethylammoniummethosulfat, Diketearoylethylhydroxyethylmethylammoniummethosulfat, Distearoylethyldimethylammoniumchlorid oder Distearoyldiethylmethylammoniumchlorid ist.

7. Zusammensetzung nach einem der vorangehenden Ansprüche, wobei das oder die kationischen Tenside, die mindestens eine Esterfunktion umfassen, in einem Gehalt von 0,1 bis 15 Gew.-%, vorzugsweise von 0,1 bis 10 Gew.-%, stärker bevorzugt von 0,1 bis 5 Gew.-%, bezogen auf das Gesamtgewicht der Zusammensetzung, vorhanden sind.

8. Zusammensetzung nach einem der vorangehenden Ansprüche, wobei die Nichtsilikonpolymere ausgewählt sind aus Polymeren mit oder ohne verknüpfte Zuckereinheiten, insbesondere aus Polysacchariden, wie Hydroxyethylcellulose und Hydroxypropylguar.

9. Zusammensetzung nach einem der vorangehenden Ansprüche, wobei das oder die Nichtsilikonpolymere in einem Gehalt von 0,01 bis 20 Gew.-%, vorzugsweise von 0,1 bis 10 Gew.-%, stärker bevorzugt von 0,2 bis 5 Gew.-%, bezogen auf das Gesamtgewicht der Zusammensetzung, vorhanden sind.

10. Zusammensetzung nach einem der vorangehenden Ansprüche, weiterhin umfassend mindestens ein Silikon mit quaternären Ammoniumgruppen.

11. Zusammensetzung nach dem vorangehenden Anspruch, **dadurch gekennzeichnet, dass** das Silikon Quaternium-80 ist.

12. Verfahren zur Behandlung der Haare bestehend aus dem Aufbringen auf die Haare von mindestens einer Haarzusammensetzung nach einem der vorangehenden Ansprüche, und anschließend gegebenenfalls im Durchführen von Ausspülen.

13. Verfahren nach dem vorangehenden Anspruch, weiterhin umfassend den Schritt bestehend aus der Durchführung einer Nachbehandlung mit den Haaren nach der Behandlung mit Hilfe einer Haarzusammensetzung nach einem der Ansprüche 1 bis 11, wobei die Nachbehandlung ausgewählt ist aus der Anwendung eines Conditioners, einer Dauerwelle, eines Entkrausungsmittels, eines Haarfärbe- oder eines Haarentfärbeprodukts.

14. Verfahren nach einem der Ansprüche 12 und 13, wobei der Behandlungsschritt der Haare durchgeführt wird unter Frottieren der Haare mit bloßen Händen oder unter Zuhilfenahme von mindestens einer Fläche zwischen den Händen und den mit der Haarzusammensetzung befrachteten Haaren.

15. Kit zur Behandlung von Haaren, umfassend:
- eine Haarzusammensetzung wie nach Anspruch 1 definiert, und
- einen Träger, umfassend Anleitungen zum Einsatz der Haarzusammensetzung auf den Haaren.

16. Kit nach dem vorangehenden Anspruch, umfassend weiterhin eine Zusammensetzung zur Nachbehandlung der Haare, die vorzugsweise ausgewählt ist aus einem Konditioner, einer Dauerwelle, einem Entkrausungsmittel, einem Haarfärbe- oder einem Haarentfärbeprodukt.

## Claims

1. A capillary composition comprising at least pumice stone particles, one or several cationic surfactants including at least one ester function and one or several non-silicone polymers, said non-silicone polymers being selected from among thickener polymers with sugar units, and said cationic surfactant including at least one ester function being selected from among quaternary ammonium salts containing at least one ester function of the following formula: 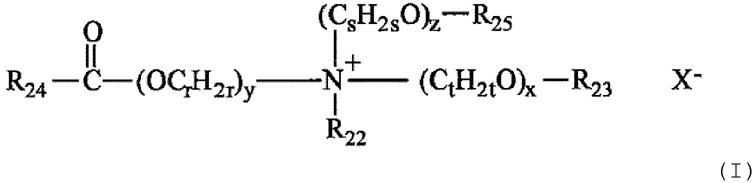 wherein:
R₂₂ is selected from among C₁-C₆ alkyl radicals and C₁-C₆ hydroxyalkyl or dihydroxyalkyl radicals;
R₂₃ is selected from among:
- the 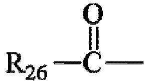 radical,
- the saturated or unsaturated, linear or branched C₁-C₂₂ hydrocarbon radicals R₂₇, and
- the hydrogen atom,
R₂₅ is selected from among:
- the 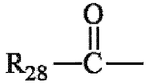 radical,
- the saturated or unsaturated, linear or branched C₁-C₂₂ hydrocarbon radicals R₂₉, and
- the hydrogen atom,
R₂₄, R₂₆ and R₂₈, either identical or different, are selected from among saturated or unsaturated, linear or branched C₇-C₂₁ hydrocarbon radicals;
r, s and t, either identical or different, are integers with a value from 2 to 6;
y is an integer of value 1 to 10;
x and z, either identical or different, are integers with values from 0 to 10; and
X⁻ is a simple or complexe, organic or inorganic anion;
with the proviso that the sum x+y+z has a value from 1 to 15, only when x has the value 0, then R₂₃ refers to R₂₇ and only when z has the value 0, then R₂₅ refers to R₂₉.

2. The composition according to claim 1, the pumice stone particles having an average diameter by volume of less than or equal to 500 µm, preferably comprised between 50 and 500 µm, better less than or equal to 300 µm, for example comprised between 100 and 300 µm.

3. The composition according to one of the preceding claims, wherein the pumice stone particles are present in a content ranging from 0.1 to 35% by weight, notably from 5% to 30%, in particular from 10% to 25% by weight, for example from 15% to 20% by weight, better from 18% to 20% by weight based on the total weight of the composition.

4. The composition according to any of the preceding claims, wherein the pumice stone particles have a hardness from 3 to 10 Mohs, or even greater than or equal to 4, for example greater than or equal to 5, and in particular ranging from 5 to 5.5 on the Mohs scale.

5. The composition according to any of the preceding claims, wherein:
R₂₂ designates a methyl or ethyl radical,
x and y are equal to 1,
z is equal to 0 or 1,
r, s and t are equal to 2,
R₂₃ is selected from among:
- the 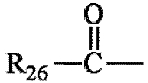 radical,
- the methyl or ethyl C₁₄-C₂₂ hydrocarbon radicals,
- the hydrogen atom;
R₂₅ is selected from among:
- the 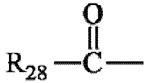 radical, and
- the hydrogen atom;
R₂₄, R₂₆ and R₂₈, either identical or different, being selected from among saturated or unsaturated, linear or branched C₁₃-C₁₇ hydrocarbon radicals, and preferably from among saturated or unsaturated, linear or branched C₁₃-C₁₇ alkyl and alkenyl radicals.

6. The composition according to any of the preceding claims, wherein the cationic surfactant, including at least one ester function is a salt of diacycloxydialkyl-hydroxyalkylammonium or a salt of diacycloxytrialkylammonium and in particular dipalmitoylethyl hydroxyethylammonium methosulfate, dicetearoylethylhydroxyethyl methylammonium methosulfate, distearoylethyldimethylammonium chloride or distearoyldiethylmethylammonium chloride.

7. The composition according to any of the preceding claims, wherein the cationic surfactant(s) including at least one ester function is(are) present in a content ranging from 0.1 to 15% by weight, preferably from 0.1% to 10% by weight, better from 0.1% to 5% by weight, based on the total weight of the composition.

8. The composition according to any of the preceding claims, wherein the non-silicone polymers are selected from associative polymers with a sugar unit or not, notably from among polysaccharides such as hydroxyethylcellulose and hydroxypropyl guar.

9. The composition according to any of the preceding claims, wherein the non-silicone polymers are present in a content ranging from 0.01 to 20% by weight, preferably from 0.1 to 10% by weight, better from 0.2 to 5% by weight based on the total weight of the composition.

10. The composition according to any of the preceding claims, further comprising at least one silicone with quaternary ammonium groups.

11. The composition according to the preceding claim, **characterized in that** said silicone is Quaternium-80.

12. A method for treating hair consisting of applying on the hair at least one capillary composition according to any of the preceding claims, and then optionally carrying out rinsing.

13. The method according to the preceding claim, further including the step of consisting of subjecting the hair to a post-treatment, after treatment by means of a capillary composition according to any of claims 1 to 11, the post-treatment being selected from application of a conditioner, of a perm, of a hair-straightener, of a hair coloring product or a bleach.

14. The method according to any of claims 12 and 13, the hair treatment step being carried out by rubbing the hair with naked hands or by interposing at least one surface between the hands and the hair loaded with capillary composition.

15. A hair treatment kit including:
- a capillary composition as defined in claim 1, and
- a support including instructions for use of the capillary composition on hair.

16. The kit according to the preceding claim, further including a hair post-treatment composition, preferably selected from among a conditioner, a perm, a hair straightener, a hair coloring product or a bleach.
